# EUROPEAN PATENT APPLICATION

(11) **EP 2 133 478 A2**
(43) Date of publication of application: **16.12.2009**
(21) Application number: 08007789.4
(22) Date of filing: 22.04.2008
(51) Int. Cl.: E03D 11/02

(54) **Apparatus for analyzing components of urine by using atr and method thereof**

(30) Priority: 27.02.2008 KR 20080017910
(71) Applicant: Jsm Healthcare Inc, Kyunggi-do 463-070 (KR)
(72) Inventor: Kim, Dong Soo, Seongnam-si Gyeonggi-do 463-500 (KR)
(74) Representative: Bertsch, Florian Oliver

(57) **Abstract**

The present invention relates to an apparatus for analyzing concentration and the like of components of urine, and more particularly, to an apparatus for analyzing concentration and the like of components of urine by using ATR-IR (Attenuated Total Reflectance Infrared Spectroscopy) and a method thereof. The apparatus for analyzing components of urine comprises a toilet bowl which has a recessed or flat-shaped urine collecting part; a piping part for guiding the urine collected from the urine collecting part; and an analyzing part for analyzing the components of the urine collected at the urine collecting part, wherein the urine collecting part is formed at an inner front side of the toilet bowl, the analyzing part comprises ATR, and the analyzing part is directly attached to the toilet bowl.

## Description

### [Technical Field]

The present invention relates to an apparatus for analyzing concentration and the like of components of urine, and more particularly, to an apparatus for analyzing concentration and the like of components of urine by using ATR-IR (Attenuated Total Reflectance Infrared Spectroscopy) and a method thereof.

### [Background Art]

Generally, a method of analyzing the components of the urine uses visible rays. Three wavelengths in a visible ray range are mainly used to analyze the components of the urine. At this time, the above-mentioned method is performed by using a urine test paper. Since the disposable test paper is continuously used in the method, a user has to periodically buy the test paper so as to perform the analyzing of the components of the urine, and it is very inconvenient to smear the urine on the test paper and also carry out the method. Further, since the user has to be equipped with the test paper and an apparatus for performing the method, it is difficult to come into wide use.

As other method which does not use the test paper, there has been proposed a spectral analysis method. In this method, an apparatus for introducing a sample from a toilet bowl is separately provided. And an apparatus for analyzing the introduced sample, a light source and a detector are constructed in a transmission type in which they are arranged in parallel (at an angle of 180°). However, in this case, the additional apparatus has to be provided, and particularly, light used in this method is a near infrared ray, and the near infrared ray used in the analyzing apparatus has a wavelength band of 800nm - 2500nm. The light having the wavelength band is suitable for the analysis of a single component contained in the urine, but in case of analyzing plural components in the urine, measured values with respect to the plural components are overlapped with each other, and thus it is difficult to analyze the components of the urine.

Therefore, it is required to provide an apparatus for conveniently and precisely analyzing the plural components and a method thereof.

Further, since there is not yet provided an apparatus for conveniently measuring blood pressure, body fat and the like as well as analyzing the urine by a simple action while the user is seated on the toilet bowl, the user has to check the components of the urine, the blood pressure, the body fat and the like at a different time and a different place.

### [Disclosure of the Invention]

### [Technical Subject]

It is an object of the present invention to provide an apparatus for analyzing components of urine, in which ATR as a component of ATR-IR (Attenuated Total Reflectance Infrared Spectroscopy)is directly attached to a toilet bowl so as to conveniently and precisely analyze the components in the urine, and a method thereof.

It is another object of the present invention to provide an apparatus for analyzing components of urine, which uses light having a wavelength of 1000 ∼ 15000nm so as to conveniently and precisely analyze the components in the urine, and a method thereof.

It is yet another object of the present invention to provide a health examination system which can simultaneously measure blood pressure, body fat and the like as well as analyze the components in the urine by a simple action.

### [Technical Solution]

To achieve the above objects, the present invention provides an apparatus for analyzing components of urine, comprising a toilet bowl which has a recessed or flat-shaped urine collecting part; a piping part for guiding the urine collected from the urine collecting part; and an analyzing part for analyzing the components of the urine introduced through the piping part, wherein the urine collecting part is formed at an inner front side of the toilet bowl, the analyzing part comprises ATR, and the analyzing part is directly attached to the toilet bowl.

Preferably, the piping part comprises an input pipe for guiding the urine and a reference material measuring a reference value to the ATR; and an output pipe for discharging the reference material and the urine from the ATR.

Preferably, the input pipe comprises a solenoid valve for controlling the reference material and the urine guided to the ATR; at least one cartridge for supplying the reference material; and a cleaning tank for supplying a cleaning solution to clean the input pipe and the ATR.

Further, to achieve the above objects, the present invention provides an apparatus for analyzing components of urine, comprising a toilet bowl which has a recessed or flat-shaped urine collecting part; and an analyzing part for analyzing the components of the urine collected at the urine collecting part, wherein the urine collecting part is formed at an inner front side of the toilet bowl, the analyzing part comprises ATR, and the analyzing part is directly attached to the toilet bowl.

Preferably, the urine collecting part is horizontally or inclinedly formed at an inner side of the toilet bowl, and the urine collecting part is formed into a hemispherical surface, and a hole is formed at a center portion of the hemispherical surface. And the analyzing part comprises a prism, and an upper surface of the prism is formed into a curved shape and coupled to the hole, or formed into a fat shape and coupled to the hole, or the upper surface of the prism is attached under the hole.

Preferably, the urine analyzing apparatus further comprises a cleaning solution supplying part which is disposed at a higher position than the urine collecting part so as to clean the urine collecting part.

Preferably, the urine analyzing apparatus further comprises an air injecting part which is disposed at a higher position than the urine collecting part so as to dry the urine collecting part. Preferably, the analyzing part comprises a light source for generating light; a monochromator for generating monochromatic light using the light; a reflecting mirror for guiding the monochromatic light to the ATR; a detector for detecting the monochromatic light passed through the ATR; a controller for measuring a property of the monochromatic light detected by the detector and also controlling the light source, the monochromator, the reflecting mirror and the detector.

Preferably, the monochromatic light has a wavelength of 1000 ∼ 15000nm, and the analyzing part is made smaller by using MEMS (Micro Electro Mechanical Systems) technology.

Further, to achieve the above objects, the present invention provides a method of analyzing components of urine, comprising the steps of measuring a spectrum of a reference material introduced through a urine collecting part; measuring an absorption spectrum of the urine introduced through the urine collecting part; obtaining a calibration curve which indicates correlation between the absorption spectrum and a standard value which is generated by previously measuring each component of the urine; estimating an amount of each component contained in the urine by using the calibration curve, wherein the urine collecting part is formed at an inner front side of a toilet bowl, the spectrum of the reference material and the spectrum of the urine are measured by using ATR, and the ATR is directly attached to the toilet bowl.

Preferably, the spectrum of the reference material and the spectrum of the urine are measured by using monochromatic light introduced to the ATR. (Preferably, the method of analyzing components of urine further comprises the step of cleaning the urine collecting part using a cleaning solution after measuring the absorption spectrum of the urine, and the cleaning solution and the reference material may be the same material. Also, the method of analyzing components of urine further comprises the step of drying the urine collecting part using an air injecting part which is disposed at a higher position than the urine collecting part.

Further, to achieve the above objects, the present invention provides a health examination system which comprises a bidet device disposed at a rear side of a toilet bowl and a body fat measuring apparatus coupled with the toilet bowl, wherein the body fat measuring apparatus comprises a handle disposed at left and right sides of the toilet bowl; eight electrodes which are respectively provided at four contact points in pairs, and wherein the two out of the four contact points are disposed at an upper surface of a seat portion of the toilet bowl, which is contacted with user's hips or femoral region, and the other two contact points are disposed at the handles.

Preferably, each of the contact points comprises a voltage electrode and a current electrode, and the handle is disposed to be inserted into a side portion of the toilet bowl, and also a cover is further provided thereon so as to prevent water from being contacted.

Preferably, the health examination system further comprises a urine analyzing apparatus for measuring components contained in urine, wherein ATR is directly attached to the toilet bowl.

Further, to achieve the above objects, the present invention provides a health examination system comprising a bidet device which is disposed at a rear side of a toilet bowl; a weight measuring apparatus for measuring user's weight using a plurality of load cells disposed under a seat portion of the toilet bowl; and a urine analyzing apparatus for measuring components contained in urine using ATR, wherein the ATR is directly attached to the toilet bowl.

Preferably, the health examination system further comprises a blood pressure measuring apparatus for measuring user's blood pressure; and a fingerprint recognition apparatus for identifying a user of the urine analyzing apparatus by recognizing user's fingerprint, wherein the blood pressure measuring apparatus and the fingerprint recognition apparatus are disposed at an arm rest on which user's arm is rested, and the user can simultaneously perform the fingerprint recognizing and the blood pressure measuring, while being seated on the toilet bowl.

Preferably, the health examination system further comprises a monitor for displaying at least one of urine analyzing information measured by the urine analyzing apparatus, weight information measured by the weight measuring apparatus, fingerprint information recognized by the fingerprint recognizing apparatus, blood pressure information measured by the blood pressure measuring apparatus and body fat information measured by the body fat measuring apparatus, wherein the monitor is disposed at the arm rest.

Preferably, the health examination system further comprises an agent injecting apparatus for supplying an agent used in the health examination system, wherein the agent injecting apparatus is inclinedly disposed at a rear side of the toilet bowl, and also the agent injecting apparatus is connected with the bidet device.

Preferably, at least one of the urine analyzing information, the weight information, the fingerprint information, the blood pressure information and the body fat information can be transferred through Internet or Ethernet, and also analyzed results using the transferred information can be received.

Further, to achieve the above objects, the present invention provides a health examination system which comprises a bidet device disposed at a rear side of a toilet bowl and an electrocardiogram measuring apparatus coupled with the toilet bowl, wherein the electrocardiogram measuring apparatus comprises two contact points disposed at left and right handles of the toilet bowl; and two contact points disposed at an upper surface of the toilet bowl, which is contacted with user's hips or femoral region, wherein each contact point has two electrodes, and the electrocardiogram measuring apparatus measures an electrocardiogram by transmitting an induced current through an user of the health examination system using the eight electrodes disposed at the four contact points and then measuring potential differences among the electrodes.

### [Effect of the Invention]

According to the urine analyzing apparatus and method, since the urine is measured by using the ATR and the Fourier-transform infrared spectroscopy which are directly attached to the toilet bowl, it is possible to precisely measure multiple components of the urine within a short time period.

Also, since the urine analyzing apparatus and method of the present invention does not need additional members like the piping part, durability thereof is increased. Further, according to the present invention, since the analyzing part of the urine analyzing apparatus is directly attached to the toilet bowl, the user can monitor the components contained in the urine every day, and thus it is possible to check his/her own health information periodically.

Also, since the urine analyzing apparatus and method of the present invention measure the components contained in the urine by using light having a wavelength of 1000 ∼ 15000nm, it is possible to quickly and precisely measure the multiple components of the urine.

In addition, since the present invention can provide a health examination system which can measure blood pressure, body fat and the like and also can analyze the components of the urine by a simple action while the user is seated on the toilet bowl, the user can conveniently and periodically measure the components of the urine, the blood pressure, the body fat and the like.

### [Brief Description of the Drawings]

Fig. 1 is a perspective view of a health examination system including an apparatus for analyzing components of urine according to an embodiment of the present invention.
Fig. 2 is a perspective view of a health examination system including an apparatus for measuring body fat according to an embodiment of the present invention.
Fig. 3 is a perspective view of a handle of the apparatus for measuring the body fat according to an embodiment of the present invention.
Fig. 4 is a view showing a current flowing direction from the apparatus for measuring the body fat according to the embodiment of the present invention.
Fig. 5 is a perspective view of an analyzing part of the apparatus for analyzing the components of the urine according to the embodiment of the present invention.
Fig. 6 is a perspective view of a piping part of the apparatus for analyzing the components of the urine according to a first embodiment of the present invention.
Fig. 7 is a perspective view of a urine collecting part formed at a toilet bowl of the apparatus for analyzing the components of the urine according to a second embodiment of the present invention.
Fig. 8 is a cross-sectional view of the urine collecting part formed at the toilet bowl of the apparatus for analyzing the components of the urine according to the second embodiment of the present invention.
Fig. 9 is a cross-sectional view of an analyzing part coupled with the urine collecting part of the apparatus for analyzing the components of the urine according to the second embodiment of the present invention.
Fig. 10 is a flow chart showing a method of analyzing the components of the urine according to an embodiment of the present invention.
Fig. 11 is a graph showing a Glucose spectrum contained in the urine, which is measured by using the apparatus for analyzing the components of the urine according to an embodiment of the present invention.
Fig. 12 is a graph showing a Creatine spectrum contained in the urine, which is measured by using the apparatus for analyzing the components of the urine according to the embodiment of the present invention.
Fig. 13 is a graph showing a Urea spectrum contained in the urine, which is measured by using the apparatus for analyzing the components of the urine according to the embodiment of the present invention.
Fig. 14 is a graph showing a Cholesterol spectrum contained in the urine, which is measured by using the apparatus for analyzing the components of the urine according to the embodiment of the present invention.
Fig. 15 is a graph showing a Bilirubin spectrum contained in the urine, which is measured by using the apparatus for analyzing the components of the urine according to the embodiment of the present invention.
Fig. 16 is a graph showing a Uric acid spectrum contained in the urine, which is measured by using the apparatus for analyzing the components of the urine according to the embodiment of the present invention.
Fig. 17 is a graph showing a Nitrite spectrum contained in the urine, which is measured by using the apparatus for analyzing the components of the urine according to the embodiment of the present invention.
Fig. 18 is a graph showing a calibration curve of Glucose contained in the urine, which is measured by using the apparatus for analyzing the components of the urine according to an embodiment of the present invention.
Fig. 19 is a graph showing a calibration curve of Creatine contained in the urine, which is measured by using the apparatus for analyzing the components of the urine according to the embodiment of the present invention.
Fig. 20 is a graph showing a calibration curve of Urea contained in the urine, which is measured by using the apparatus for analyzing the components of the urine according to the embodiment of the present invention.
Fig. 21 is a graph showing a calibration curve of Cholesterol contained in the urine, which is measured by using the apparatus for analyzing the components of the urine according to the embodiment of the present invention.
Fig. 22 is a graph showing a calibration curve of Bilirubin contained in the urine, which is measured by using the apparatus for analyzing the components of the urine according to the embodiment of the present invention.
Fig. 23 is a graph showing Uric acid in the urine sample, which is measured by using the apparatus for analyzing the components of the urine according to the embodiment of the present invention.
Fig. 24 is a graph showing Urea in the urine sample, which is measured by using the apparatus for analyzing the components of the urine according to the embodiment of the present invention.

### [Best mode for the Invention]

If at all possible, terms used in the description were selected from general terms widely used in the art. However, in particular cases, some terms which were optionally selected by the applicant were used, and the meanings thereof are defined in the description. Thus, the present invention should be construed by the defined meanings thereof but not the meanings of the terms themselves.

Practical and presently preferred embodiments of the present invention are illustrative as shown in the following Examples and Comparative Examples.

However, it will be appreciated that those skilled in the art, on consideration of this disclosure, may make modifications and improvements within the spirit and scope of the present invention.

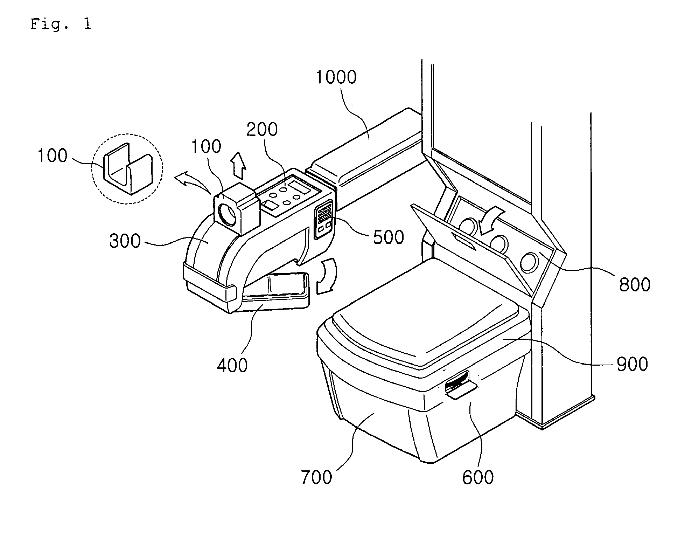

Fig. 1 is a perspective view of a health examination system including an apparatus for analyzing components of urine according to an embodiment of the present invention. Referring to Fig. 1, the health examination system includes an apparatus 100 for measuring blood pressure (hereinafter, called "blood pressure measuring apparatus"), a bidet controlling apparatus 200, a fingerprint recognition apparatus 300, a monitor 400, a main controlling apparatus 500, an apparatus 600 for measuring body fat body fat measuring apparatus (hereinafter, called "body fat measuring apparatus"), an apparatus 700 for analyzing components of urine (hereinafter, called "urine analyzing apparatus"), an agent injecting apparatus 800 and a weight measuring apparatus 900.

In the health examination system, the blood pressure can be measured by using the blood pressure measuring apparatus 100. The blood pressure measuring apparatus 100 may be constructed as follows. In case that the blood pressure measuring apparatus 100 is not used, the blood pressure measuring apparatus 100 can be inserted into an arm rest 1000, and in case that a user is seated on an toilet bowl and then pushes a 'blood pressure measuring' button of the main controlling apparatus 500 to measure the blood pressure, a cuff of the blood pressure measuring apparatus 100 is protruded upward. Then, after the user's arm is inserted into the cuff, the blood pressure is measured by pushing a 'start' button. The monitor 400 displays a pulse rate and/or a maximum/minimum value of the blood pressure. If the blood pressure measuring is completed, the user pulls out his/her arm from the cuff, and pushes again the 'blood pressure measuring' button. Then, the blood pressure measuring apparatus 100 is automatically inserted into the arm rest 1000. The blood pressure measuring apparatus 100 may be formed into various shapes like an opened cuff shown in a circle of the drawing, by which the blood pressure is automatically measured, if the user puts his/her wrist thereon. In this case, it is advantage to make it smaller comparing with a rectangular parallelepiped type, since a portion in which the arm is inserted can be made smaller.

In Fig. 1, the blood pressure measuring apparatus 100 is formed into the rectangular parallelepiped type or the opened cuff type and disposed on an upper surface of the arm rest 1000. However, the present invention is not limited to such the type and position.

If a fingerprint of the user is contacted with the fingerprint recognition apparatus 300 while the user is seated on the toilet bowl, the user who is being seated on the toilet bowl is identified by the fingerprint recognition apparatus 300. Since one of the conventional fingerprint recognition apparatuses 300 may be used, the detailed description thereof will be omitted.

Furthermore, in the health examination, a user's weight can be measured by using the weight measuring apparatus 900, and body fat can be also measure by using the body fat measuring apparatus 600.

The weight measuring apparatus 900 may be formed by disposing four load cells at a lower side of a seat portion of the toilet bowl. When the user is seated on the toilet bowl, the measuring is automatically started, and a measured value is displayed on the monitor 400 which is disposed at the arm rest 1000.

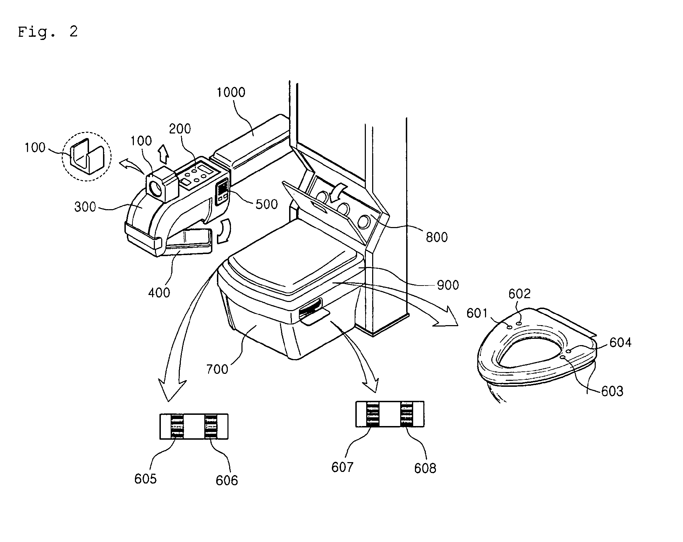

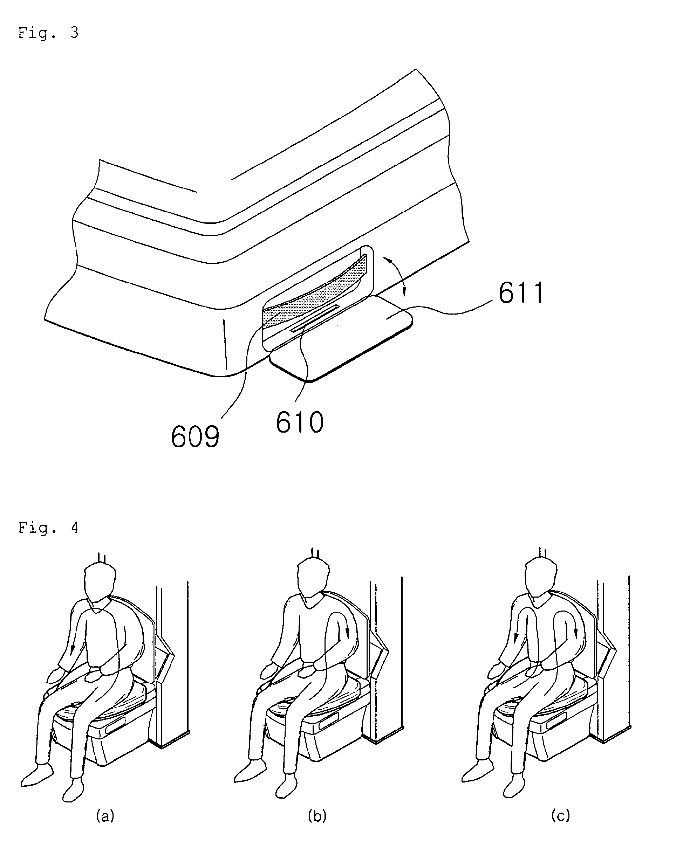

Further, if the user grasps a handle of the body fat measuring apparatus 600, which is disposed in a left/right side of an upper portion of the toilet bowl, after the user is seated on the toilet bowl, the body fat measuring is started. Referring to Figs. 2 and 3, the body fat measuring apparatus 600 will be described in detail.

In a method of measuring the body fat, if the user pushes the 'fat body measuring' button of the main controlling apparatus 500, a pressure sensor of the weight measuring apparatus 900 is activated so as to measure the weight. Then, the user pushes the 'start' button and stretches out both arms downwardly and grasps the handle of the body fat measuring apparatus 600. When the body fat measuring is completed, necessary information such as body fat rate, muscle mass and the like is calculated and displayed on the monitor 400 by using previously stored user's information such as age, sex, height and weight. If the weight information is previously acquired, the process of measuring the weight may be omitted.

The monitor 400 may be constructed so as to be horizontally pivoted around a shaft which is fixed to a lower surface of the arm rest 1000. After the monitor 400 is protruded, it can be vertically pivoted again around other shaft so as to be positioned at a proper angle that the user can comfortably see the monitor 400.

In Fig. 1, in order to measure the body fat, the handle of the body fat measuring apparatus 600 is horizontally disposed at the left and right sides of the toilet bowl. However, the body fat measuring apparatus 600 is not limited to such the shape and position, and the button of the main controlling apparatus 500 for measuring the body fat is not also limited to a particular type.

In addition, the health examination system can measure and display sugar, protein, blood and the like contained in the urine by using the urine analyzing apparatus 700. The urine analyzing apparatus 700 will be described in detail with reference to Figs. 2 to 6.

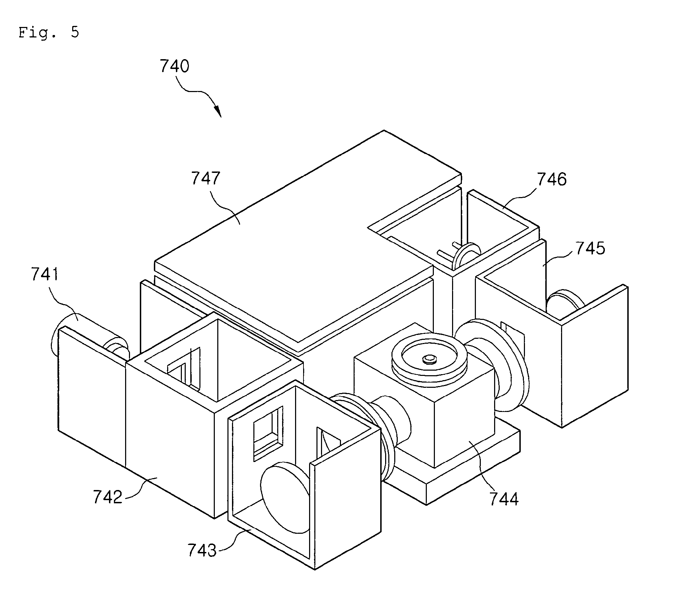

Moreover, the health examination system includes the agent injecting apparatus 800 which is disposed at a rear side of the urine analyzing apparatus 700. The agent injecting apparatus 800 can store various agents such as cleansing agent (e.g., vagina cleansing agent), aromatic agent and the like. The agent injecting apparatus 800 may be constructed to be directly and exactly coupled with an agent case and also to be slightly inclined so that the agent in the case can be facilely flowed down. Therefore, the agent case itself can be coupled to the agent injecting apparatus 800, and if the agent in the case is completely consumed, the agent case can be removed from the agent injecting apparatus 800. The agent injecting apparatus 800 is connected with a bidet device (shown in Fig. 5), and the agent in the agent injecting apparatus 800 is ejected through the bidet device.

Furthermore, the health examination system includes a separate memory (not shown), and the memory stores measured data (e.g., weight, body fat rate, sugar, protein and the like) for each user. And also, using Ethernet or Internet network, the health examination system can transfer the measured data stored in the memory to other place like a medical center and then receive analyzed results based on the transferred data. In addition, while using the health examination system, the user can see a movie or use Internet Services through the monitor 400 and also use a built-in telephone (not shown).

Fig. 2 is a perspective view of the health examination system including the body fat measuring apparatus according to an embodiment of the present invention. Referring to Fig. 2, the body fat measuring apparatus 600 includes four electrodes 601, 602, 603 and 604 disposed at the seat portion of the toilet bowl and other four electrodes 605, 606, 607 and 608 disposed at both handles. Therefore, the body fat measuring apparatus 600 measures the body fat using the eight electrodes.

In other words, a voltage electrode and a current electrode are disposed at each of the left and right handles, and also four electrodes (two voltage electrodes and two current electrodes) are additionally disposed at the seat portion of the toilet bowl, which is contacted with user's hips or femoral region. The two electrodes (voltage and current electrodes) forms one contact point.

Fig. 3 is a perspective view of the handle of the body fat measuring apparatus according to an embodiment of the present invention. Referring to Fig. 3, the handle 609 may be disposed to be inserted into a side portion of the toilet bowl, and also a cover 611 may be further provided thereon so as to prevent water from being contacted. Furthermore, a slit 610 may be formed at a lower side of the handle 609 so that water seeped through an outer edge can be drawn off there through. The cover and slit may have various shapes, respectively.

Fig. 4 is a view showing a current flowing direction from the body fat measuring apparatus according to the embodiment of the present invention. Referring to Fig. 4, as shown in (c) of Fig. 4, if the user is seated on the toilet bowl and grasps both handles in order to measure the body fat, voltage and current are supplied through two different paths so as to measure body impedance. Thus, it is possible to precisely measure the body fat.

In a conventional four-electrode type, as shown in (a) and (b) of Fig. 4, since the right or left arm is measured through the left abdominal region, in any case, only a half of the upper part of the body is measured. Thus, it is not possible to measure the impedance of the whole upper part of the body.

However, according to the body fat measuring apparatus 600 of the present invention, firstly, the current is flowed from the voltage and current electrodes 603 and 604 through the left hip (or femoral region), the abdominal region and the left arm to the voltage and current electrodes 607 and 608.

Secondly, the current is flowed from the voltage and current electrodes 601 and 602 through the right hip (or femoral region), the abdominal region and the right arm to the voltage and current electrodes 605 and 606.

Therefore, since it is possible to measure the impedances of the left and right upper parts of the body including the abdominal region, it is possible to precisely measure the impedance of the whole upper part of the body including the abdominal region. Further, since the body fat rate is calculated on the basis of the measured value, it is possible to more precisely measure the body fat comparing with the conventional four-electrode type.

And the health examination system of the present invention further includes an electrocardiogram measuring apparatus. The electrocardiogram measuring apparatus can measure an electrocardiogram using the same electrodes used in the body fat measuring apparatus 600, but the present invention is not limited to this. That is, the electrocardiogram measuring apparatus may use the four electrodes disposed at the left and right sides of the seat portion of the toilet bowl and the four electrodes disposed at the both handles, or may use another electrodes separated from the body fat measuring apparatus.

In a method of measuring the electrocardiogram, if the user is seated and grasps the both handles, the electrocardiogram measuring apparatus transmits an induced current six times using the eight electrodes, and then measures potential differences among the electrodes.

Firstly, the potential differences among the right hand, left hand and left femoral region (or hip) are measured. That is, the potential difference between the left and right hands is measured by using the first induced current, the potential difference between the left femoral region and the right hand is measured by using the second induced current, and the potential difference between the left femoral region and the left hand is measured by using the third induced current.

Then, the potential differences among the right hand, left hand and right femoral region (or hip) are measured. That is, the potential difference between the left and right hands is measured by using the fourth induced current, the potential difference between the right femoral region and the right hand is measured by using the fifth induced current, and the potential difference between the right femoral region and the left hand is measured by using the sixth induced current.

The results measured by the above method are transferred to the main controlling apparatus 500 or the separate electrocardiogram measuring apparatus. As the result, if the measured potential difference is a positive (+) value, it is represented upward, and if the measured potential difference is a negative (-) value, it is represented downward. And then a final electrocardiogram is obtained by calculating a weighted average value of the six measured results. Hereinafter, the urine analyzing apparatus 700 will be described fully.

Fig. 5 is a perspective view of an analyzing part of the urine analyzing apparatus according to the embodiment of the present invention. Referring to Fig. 5, the urine analyzing apparatus includes a toilet bowl, a urine collecting part, an analyzing part and so on. The analyzing part may be ATR-IR (Attenuated Total Reflectance Infrared Spectroscopy), and the ATR-IR includes a light source 741, a monochromator 742, a reflecting mirror 743, 745, ATR 744, a detector 746 and a controller 747.

If a sample (e.g., urine) is introduced into the ATR 744 of the analyzing part 740, light generated from the light source 741 is guided to the monochromator 742, and the light source 741 generates the light having a wavelength of 1000 ∼ 15000nm.

The monochromator 742 monochromatizes the light generated from the light source 741 to each wave member. At this time, the monochromator 742 generates monochromatic light. The monochromator 742 may generate the monochromatic light using Fourier transform or other ways. The present invention is not limited to such the method of generating the monochromatic light. The generated monochromatic light is guided through the reflecting mirror 743, 745 to the ATR 744.

In the ATR 744, the monochromatic light passes through the sample. According to the present invention, the ATR 744 is to obtain an infrared spectrum of the sample which is difficult to be treated in the general absorption spectroscopy. And the ATR 744 means an apparatus or method which can be properly used for measuring a sample such as solid body having low solubility, film, fiber, paste, adhesive and/or powder.

When the light passes from a dense medium to a sparse medium, the light is typically reflected. At this time, a reflecting rate of the incident light becomes increased, according as an incident angle is increased. If the incident angle is greater than a certain critical angle, total reflection of the light is occurred.

When the total reflection is occurred, it is experimentally and theoretically known that the light acts as if the light is penetrated through the sparse medium in a small distance. At this time, a penetration depth of the light is changed from a few tenths to a few wavelengths.

A final penetration depth depends on a wavelength of incident light, refractive indices of the two materials and an incident angle with respect to an interface. The penetrated radiant light is called an "evanescent wave". If the sparse medium absorbs the evanescent wave, the light having an absorption band wavelength is attenuated. The ATR 744 uses an absorption principle which is occurred by the reflection at the interface between the mediums.

The light passing through the ATR 744 is reflected by the reflecting mirror 745 and then introduced to the detector 746. The light detected from the detector 746 is converted into a digital signal and then measured at the controller 747. The controller 747 measures detected data and electrically controls each part.

The analyzing part shown in Fig. 5 is an example, and the present invention is not limited to such type of analyzing part. And the present invention includes the analyzing part formed by using MEMS (Micro Electro Mechanical Systems) technology.

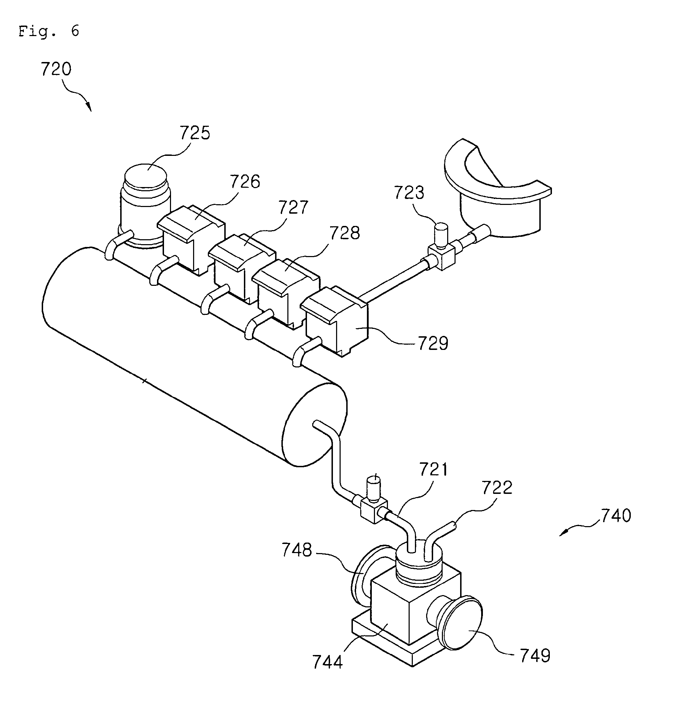

Fig. 6 is a perspective view of a piping part of the urine analyzing apparatus according to a first embodiment of the present invention. The urine analyzing apparatus includes a urine collecting part (not shown) which is formed to be recessed in the toilet bowl, a piping part 720 for introducing the urine collected from the urine collecting part to an analyzing part, and the analyzing part 740 for analyzing the urine introduced from the piping part.

Referring to Fig. 6, the piping part 720 is a unit for introducing the urine sample to the ATR 744, and the piping part 720 can be constructed as a flow type in which the urine is introduced in a liquid state. In the flow type piping part 720, firstly, while a solenoid valve 723 is in the off state, a reference material is provided through an input pipe 721 to the ATR 744 so that the analyzing part 740 measures a reference valve. Then, the reference material is discharged through an output pipe 722.

Moreover, the piping part 720 provides the urine sample to the ATR 744 through the input pipe 721 for a predetermined time period, while the solenoid valve 723 is in the on state, and the analyzing part 740 measures the sample. Then, the sample is discharged through the output pipe 722. According to the present invention, the flow type piping part 720 is attached to a conventional ATR so that the sample can be more facilely measured.

After measuring the sample, a cleaning solution is supplied from a cleaning tank 725 so as to clean the piping part 720 including the input pipe 721 and the output pipe 722. Especially, the piping part 720 includes at least one cartridge 726, 727, 728 for supplying the reference material. Therefore, various kinds of reference materials can be used, and the cartridge is constructed to be facilely replaced with new one. The reference material contains other components of the sample except a target component to be measured, and thus it is possible to minimize interference by the components except the target component.

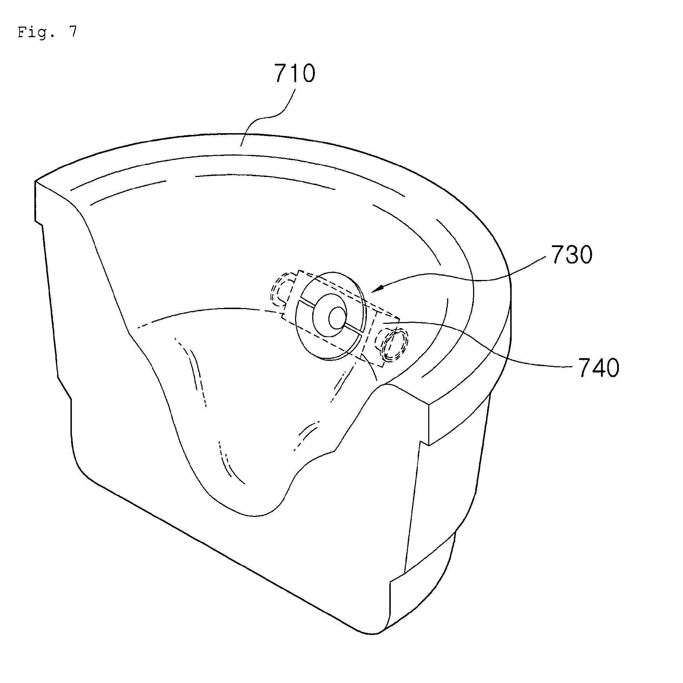

Fig. 7 is a perspective view of a urine collecting part formed at a toilet bowl of the urine analyzing apparatus according to a second embodiment of the present invention. Referring to Fig. 7, the urine analyzing apparatus includes a urine collecting part 730 for collecting the urine, a toilet bowl 710 including the urine collecting part 730, and an analyzing part 740 for analyzing the urine collected from the urine collecting part 730.

The toilet bowl 710 and the analyzing part 740 shown in Fig. 7 have the same or similar function to those in Fig. 6, and thus the description thereof will be omitted. A difference from the urine analyzing apparatus shown in Fig. 6 is that the piping part is not included. In the urine analyzing apparatus shown in Fig. 7, the urine collecting part 730 is directly connected to the analyzing part 740. Therefore, the reference material and/or the sample is introduced to the analyzing part 740 through the urine collecting part 730, and then discharged through the urine collecting part 730.

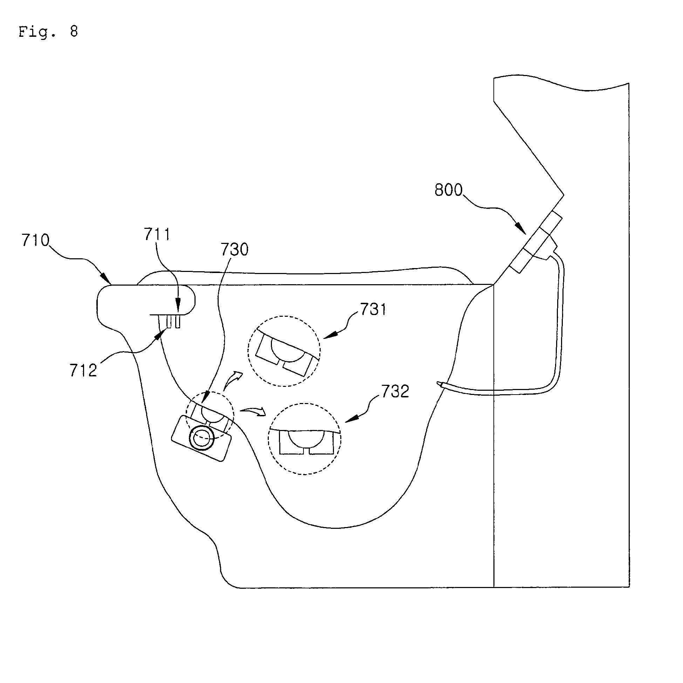

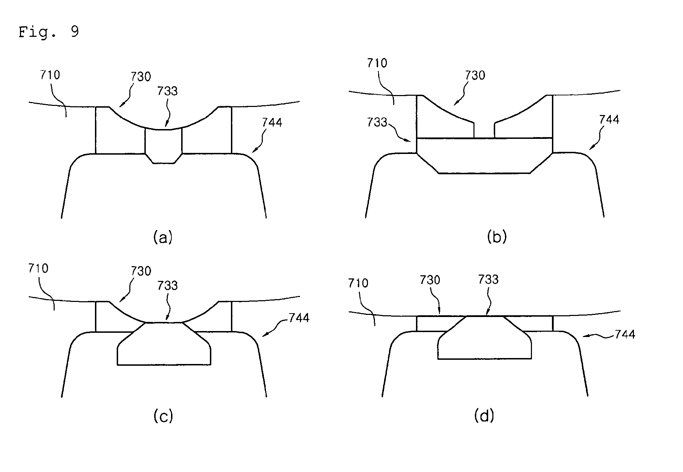

Since the urine analyzing apparatus according to the second embodiment of the present invention does not include the piping part 720, it is possible to simply measure the components of the urine without additional cost like replacement of the pipes, and it is possible to minimize influence of contaminant in the pipes. And also, right after measuring the reference value of the reference material at the same time as the cleaning process with the cleaning solution, it is possible to measure the urine. With reference to Figs. 8 and 9, the structure of the urine collecting part 730 will be described fully.

Fig. 8 is a cross-sectional view of the urine collecting part formed at the toilet bowl of the urine analyzing apparatus according to the second embodiment of the present invention. Referring to Fig. 8, the urine collecting part 730 of the urine analyzing apparatus is formed to be inclined at an inner side of a toilet bowl 710. Therefore, the urine and/or the reference material can be easily flowed and filled into the urine collecting part 730, and the urine collecting part 730 can be easily cleaned with the cleaning solution. The present invention further includes the urine collecting part 732 which is formed to be horizontally with respect to the toilet bowl 710 as well as the urine collecting part 731 which is formed to be inclined at the inner side of the toilet bowl 710.

The urine collecting part 730 can be formed into a recessed shape (e.g., a hemispherical shape) in the toilet bowl 710, and also can have a hole at a center portion thereof, in which the urine is filled. In Fig. 8, the hemispherical-shaped urine collecting part 730 is shown, but the present invention is not limited to this shape.

Further, the urine analyzing apparatus includes a cleaning solution supplying part 712 and an air injecting part 711. The cleaning solution supplying part 712 functions to supply the cleaning solution to clean the urine collecting part 730, and the air injecting part 711 functions to inject air to the urine collecting part 730 so as to dry the urine collecting part 730. According to the present invention, the same material as the reference material or other materials different from the reference material may be used as the cleaning solution.

As described above, the urine analyzing apparatus is a part of the health examination system. In Fig. 8, the agent injecting apparatus 800 which is included in the health examination system is described. The agent supplied from the agent injecting apparatus 800 is transmitted to the bidet device.

Fig. 9 is a cross-sectional view of the analyzing part coupled with the urine collecting part of the urine analyzing apparatus according to the second embodiment of the present invention. Referring to (a) of Fig. 9, the urine collecting part 730 may be formed to be coupled with a prism 733 of the ATR 744 and also to have the hemispherical shape. In other words, the prism 733 of the ATR 744 is coupled with the hole formed near the center portion of the urine collecting part 730 so that an upper surface of the prism 733 forms a part of the hemispherical shape of the urine collecting part 730. In this case, it is prevented that the urine or other materials are soaked through the interface between the urine collecting part 730 and the ATR 744 and thus the ATR 744 is contaminated.

Referring to (b) of Fig. 9, the upper surface of the prism 733 is attached under the hole formed near the center portion of the urine collecting part 730. In this case, the urine can be easily filled in the hole, and also the prism 733 can be facilely coupled with the urine collecting part 730.

Referring to (c) of Fig. 9, the prism 733 having a flat upper surface is coupled with the urine collecting part 730. And Referring to (d) of Fig. 9, the prism 733 having the flat upper surface is coupled with the urine collecting part 730 which is formed to be flat. The present invention is not limited to the coupling structure between the urine collecting part 730 and the prism 733, as shown in Fig. 9, but may have various structures.

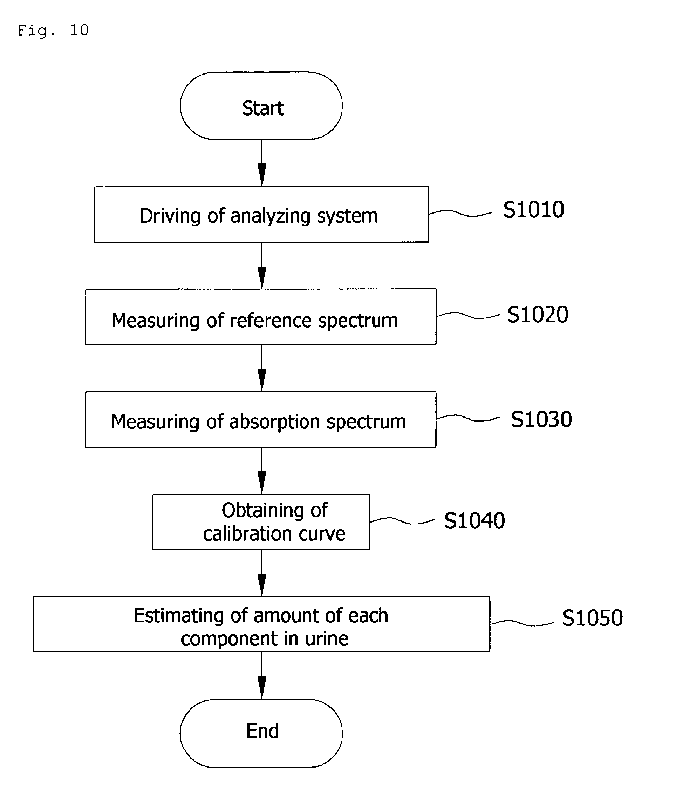

Fig. 10 is a flow chart showing a method of analyzing the components of the urine according to an embodiment of the present invention. Referring to Fig. 10, first of all, an analyzing system including the analyzing part 740 of the urine measuring apparatus of the present invention is driven (S1010). Then the reference material is introduced into the analyzing part 740, and the analyzing part 740 measures the reference spectrum (S1020). The reference material comprises water.

Then, the urine sample is directly introduced to the ATR 744 through the urine collecting part 730 of the toilet bowl 710, and the analyzing part 740 including the ATR 744 and the Fourier transform monochromator 742 measures the absorption spectrum using the introduced sample (S1030). The absorption spectrum indicates the wavenumber which is further absorbed than the reference material, comparing with the reference spectrum. An algebraic expression is calculated by -log (reference spectrum/sample spectrum).

The calibration curve indicating a correlation between the absorption spectrum and the reference value which previously measures each component of the sample is obtained (S1040). And if the absorption spectrum of the sample is assigned to the calibration curve, each value of the components contained in the urine can be estimated (S1050). Generally, after the correlation is confirmed by using standard components of urine and actual values, and R^2 and SEC(Standard Error of Calibration) as statistical indices are checked, the calibration curve is previously input to a computer.

Such an entire process is called 'routine analysis'. SEP (Standard Error of Prediction) as a statistical index, which indicates a difference between the measured value and the actual value, is the most important value in the routine analysis, and the SEP can be simultaneously obtained with the measuring process.

In other words, the correlation between the standard value which measures each component (e.g., Glucose, Albumin, Nitrite, Bilirubin, etc.) of the urine and the absorption spectrum is expressed by the calibration curve. The R^2, SEC and SEP are indices indicating that the correlation is good or bad. When the standard value and the spectrum value are expressed by an arbitrary line, the R^2, SEC and SEP indicate the correlation between the standard value and the absorption spectrum depending on a fact how much data of the two values are close to a certain line.

In case of the ideal state, i.e., in case that the correlation between the standard value and the absorption spectrum is the best, statistically, the R^2 is 1, and the SEC and SEP become close to 0. The correlation between the standard value and the absorption spectrum can be indicated by MLR (Multiple Linear Regression) or PLSR (Regression of Partial Least Square).

Using the calibration curve, the component value like glucose contained in the sample is measured. The component value is indicated by RMSEP (Root Mean of Standard Error Prediction) which is reliability significance. By measuring each component of the sample within the reliability significance, each of the components of the sample can be measured.

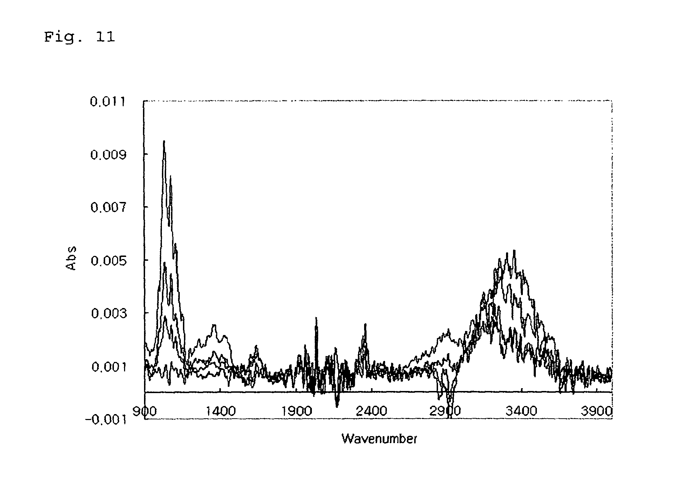

Fig. 11 is a graph showing a glucose spectrum contained in the urine according to an embodiment of the present invention, and Fig. 11 shows measured spectrum with respect to glucose concentrations of 20%, 10%, 5% and 0.2%. After water as the reference material is firstly measured, the absorption spectrum of glucose with respect to the reference material is indicated. An intensity of the spectrum is expressed by AU (Absorbance unit) value which is an absorbance of Y axis. The absorption spectrum measured by the ATR-IR is 0.01AU, and the absorption spectrum of glucose is found at a wavenumber of 900 ∼ 1400 out of a measuring wavenumber range of 900 ∼ 4000. When the glucose concentration of 20% is gradually reduced by 0.2%, the absorption spectrum is also reduced.

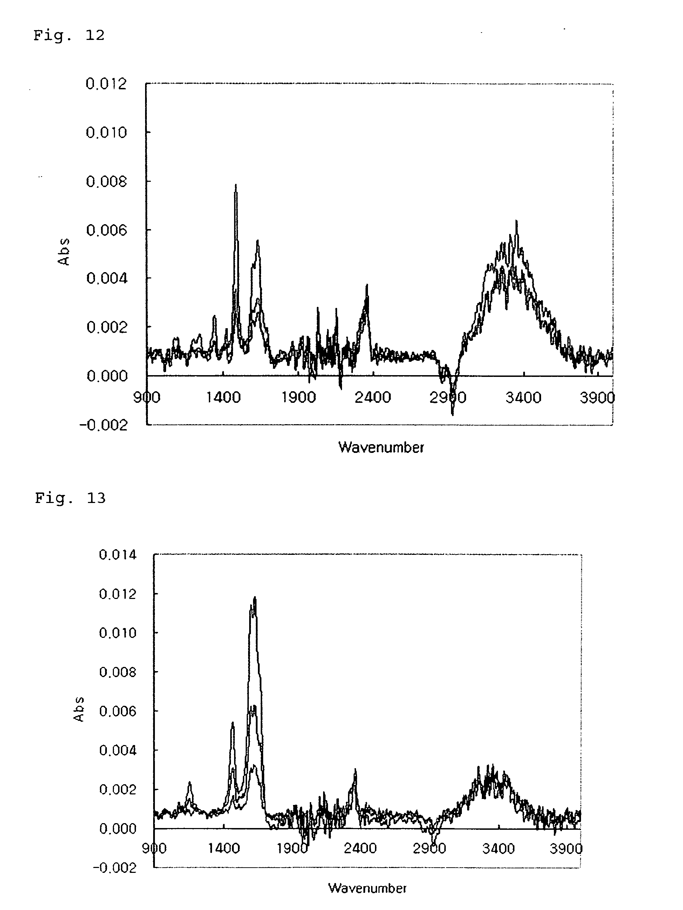

Fig. 12 is a graph showing a creatine spectrum contained in the urine according to the embodiment of the present invention, and Fig. 12 shows measured spectrum with respect to creatine concentrations of 5%, 2% and 1%. The measured spectrum is also the absorption spectrum in which the creatine is measured by using the water as the reference material. The absorption spectrum measured by the ATR-IR is shown at about 0.008AU, and the absorption spectrum of creatine is found at a wavenumber of 1400 ∼ 1900 out of the measuring wavenumber range of 900 ∼ 4000. When the creatine concentration of 5% is gradually reduced by 1%, the absorption spectrum is also reduced.

Fig. 13 is a graph showing a urea spectrum contained in the urine according to the embodiment of the present invention, and Fig. 13 shows the measured spectrum with respect to urea concentrations of 10%, 5% and 2%. The measured spectrum is also the absorption spectrum in which the urea is measured by using the water as the reference material. The absorption spectrum measured by the ATR-IR is shown at about 0.012AU, and the absorption spectrum of urea is found at a wavenumber of 1400 ∼ 1900 out of the measuring wavenumber range of 900 ∼ 4000, which is similar to the creatine of Fig. 9. When the urea concentration of 10% is gradually reduced by 2%, the absorption spectrum is also reduced.

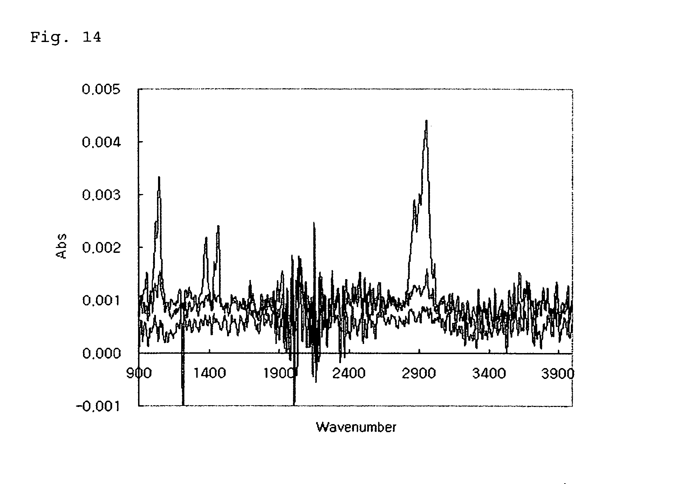

Fig. 14 is a graph showing a cholesterol spectrum contained in the urine according to the embodiment of the present invention, and Fig. 14 shows the measured spectrum with respect to cholesterol concentrations of 2%, 1% and 0.5%. The measured spectrum is also the absorption spectrum in which the cholesterol is measured by using chloroform (CHCl₃) as the reference material. The absorption spectrum measured by the ATR-IR is shown at about 0.005AU, and the absorption spectrum of cholesterol is found at a wavenumber of 2700 ∼ 3100 out of the measuring wavenumber range of 900 ∼ 4000. When the cholesterol concentration of 2% is gradually reduced by 0.5%, the absorption spectrum is also reduced.

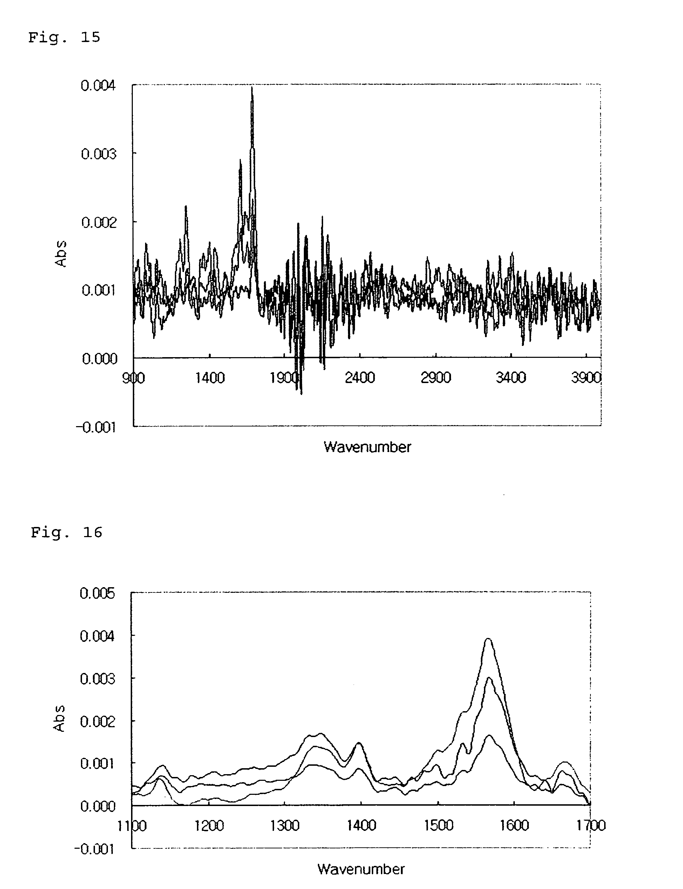

Fig. 15 is a graph showing a Bilirubin spectrum contained in the urine according to the embodiment of the present invention, and Fig. 15 shows the measured spectrum with respect to Bilirubin concentrations of 2%, 1% and 0.5%. The measured spectrum is also the absorption spectrum in which the Bilirubin is measured by using chloroform (CHCl₃) as the reference material. The absorption spectrum measured by the ATR-IR is shown at about 0.004AU, and the absorption spectrum of Bilirubin is found at a wavenumber of 1300 ∼ 1800 out of the measuring wavenumber range of 900 ∼ 4000. When the Bilirubin concentration of 2% is gradually reduced by 0.5%, the absorption spectrum is also reduced.

Fig. 16 is a graph showing a uric acid spectrum contained in the urine according to the embodiment of the present invention, and Fig. 16 shows the measured spectrum with respect to uric acid concentrations of 2%, 1% and 0.5%. The measured spectrum is also the absorption spectrum in which the uric acid is measured by using water and sodium hydroxide (NaOH) as the reference material. The absorption spectrum measured by the ATR-IR is shown at about 0.005AU, and the absorption spectrum of uric acid is found at a wavenumber of 1100 ∼ 1700 out of the measuring wavenumber range of 900 ∼ 4000. When the uric acid concentration of 2% is gradually reduced by 0.5%, the absorption spectrum is also reduced.

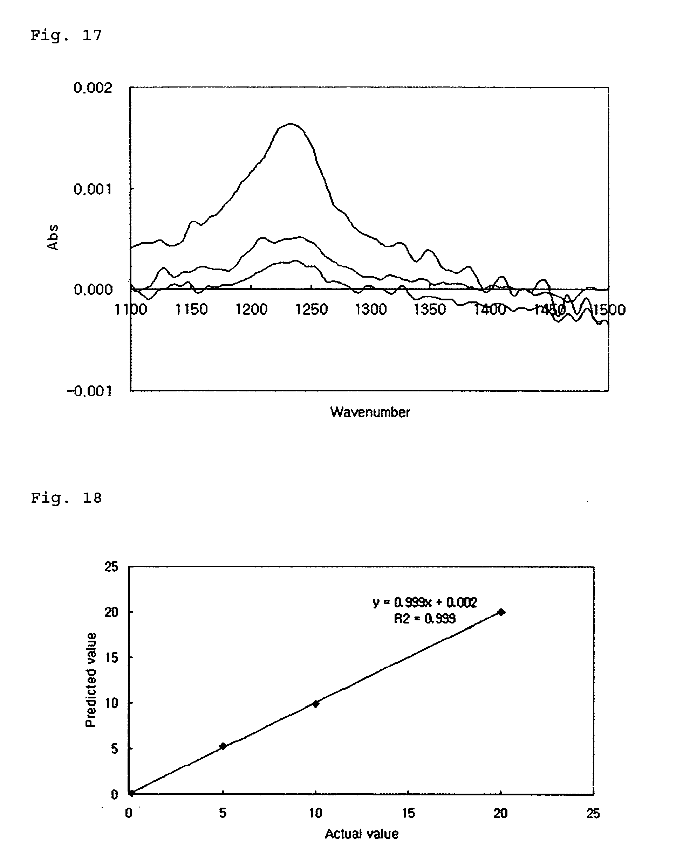

Fig. 17 is a graph showing a nitrite spectrum contained in the urine according to the embodiment of the present invention, and Fig. 17 shows the measured spectrum with respect to nitrite concentrations of 2%, 1% and 0.5%. The measured spectrum is also the absorption spectrum in which the nitrite is measured by using the water as the reference material. The absorption spectrum measured by the ATR-IR is shown at about 0.002AU, and the absorption spectrum of nitrite is found at a wavenumber of 1100 ∼ 1500 out of the measuring wavenumber range of 900 ∼ 4000, which is similar to the uric acid of Fig. 16. When the nitrite concentration of 2% is gradually reduced by 0.5%, the absorption spectrum is also reduced.

Fig. 18 is a graph showing a calibration curve of glucose contained in the urine according to an embodiment of the present invention. As shown in Fig. 18, in the correlation between change of the glucose absorption spectrum for each concentration of 20%, 10%, 5% and 0.2% and the concentration standard value, since the correlation with the absorption spectrum is indicated linearly, in which the R^2 is 0.999, it is possible to estimate an amount of glucose using the absorption spectrum.

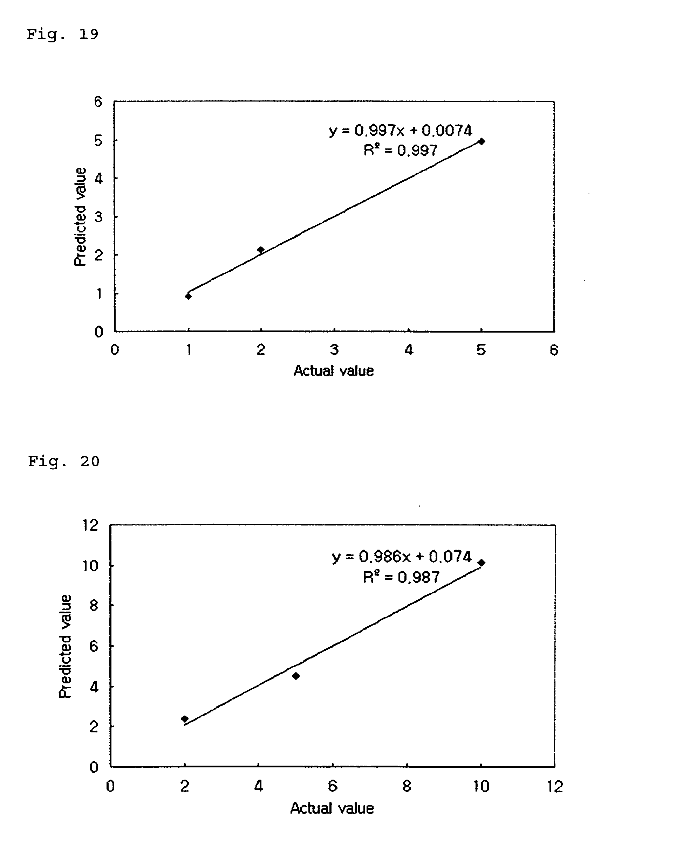

Fig. 19 is a graph showing a calibration curve of creatine contained in the urine according to the embodiment of the present invention. As shown in Fig. 19, in the correlation between change of the creatine absorption spectrum for each concentration of 5%, 2% and 1% and the concentration standard value, since the correlation with the absorption spectrum is indicated linearly, in which the R^2 is 0.997, it is possible to estimate an amount of creatine using the absorption spectrum.

Fig. 20 is a graph showing a calibration curve of urea contained in the urine according to the embodiment of the present invention. As shown in Fig. 20, in the correlation between change of the urea absorption spectrum for each concentration of 10%, 5% and 2% and the concentration standard value, since the correlation with the absorption spectrum is indicated linearly, in which the R^2 is 0.987, it is possible to estimate an amount of urea using the absorption spectrum.

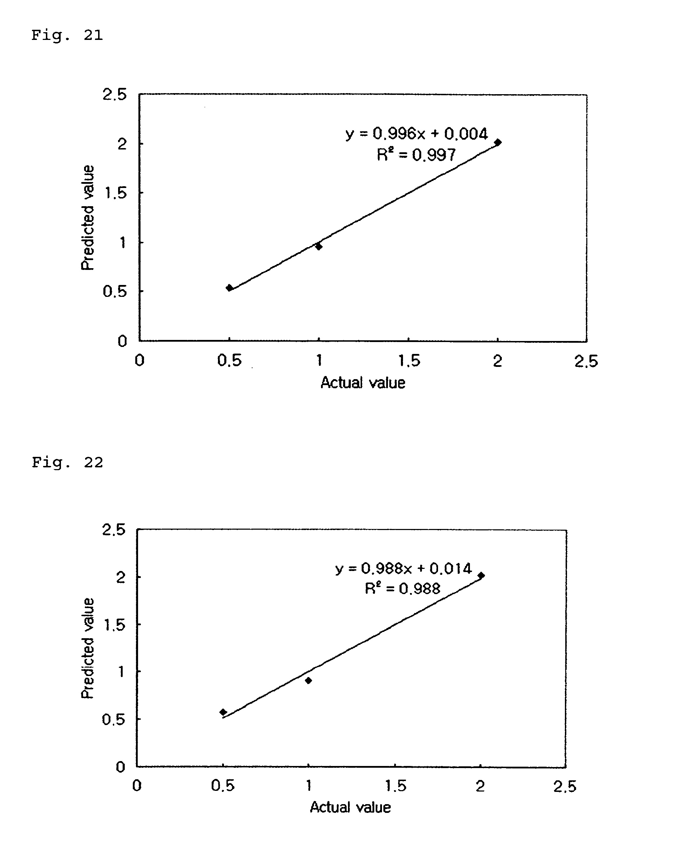

Fig. 21 is a graph showing a calibration curve of cholesterol contained in the urine according to the embodiment of the present invention. As shown in Fig. 21, in the correlation between change of the cholesterol absorption spectrum for each concentration of 2%, 1% and 0.5% and the concentration standard value, since the correlation with the absorption spectrum is indicated linearly, in which the R^2 is 0.997, it is possible to estimate an amount of cholesterol using the absorption spectrum.

Fig. 22 is a graph showing a calibration curve of Bilirubin contained in the urine according to the embodiment of the present invention. As shown in Fig. 22, in the correlation between change of the Bilirubin absorption spectrum for each concentration of 2%, 1% and 0.5% and the concentration standard value, since the correlation with the absorption spectrum is indicated linearly, in which the R^2 is 0.988, it is possible to estimate an amount of Bilirubin using the absorption spectrum.

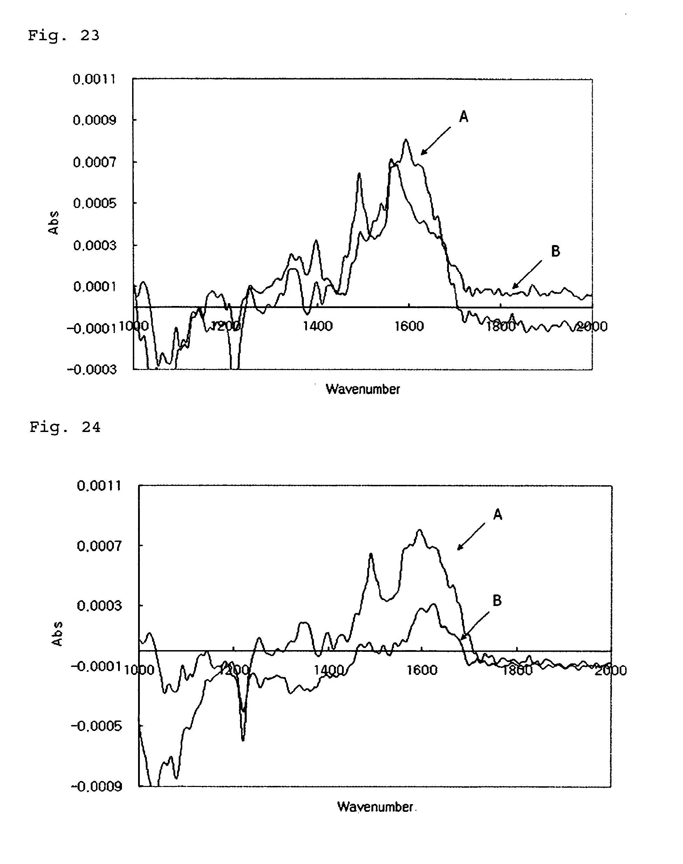

Fig. 23 shows absorption spectrums for measuring uric acid in the urine sample according to the embodiment of the present invention. As shown in Fig. 23, the absorption spectrum A shows the spectrum of uric acid which is measured after the entire sample is measured by using water as the reference material. If the concentrations of the components are the same, it is difficult to separate the absorption spectrum of uric acid due to the other components like the creatine. However, in the absorption spectrum B, the absorption spectrum is measured by using the urine excluding the uric acid as the reference material in order to separately divide the absorption spectrum of uric acid. In this case, the other absorption spectrums of creatine and the like are excluded, and only the absorption spectrum of uric acid is found.

Fig. 24 shows absorption spectrums for measuring Urea in the urine sample according to the embodiment of the present invention, which are measured by the same ways as in Fig. 23. As shown in Fig. 24, the absorption spectrum A shows the spectrum of urine which is measured after the entire sample is measured by using water as the reference material. If the concentrations of the components are the same, it is difficult to separate the absorption spectrum of urine due to the other components like the creatine. However, in the absorption spectrum B, the absorption spectrum is measured by using the urine excluding the urine as the reference material in order to separately divide the absorption spectrum of urine. In this case, the other absorption spectrums of creatine and the like are excluded, and only the absorption spectrum of urine is found.

Those skilled in the art will appreciate that the conceptions and specific embodiments disclosed in the foregoing description may be readily utilized as a basis for modifying or designing other embodiments for carrying out the same purposes of the present invention.

Those skilled in the art will also appreciate that such equivalent embodiments do not depart from the spirit and scope of the invention as set forth in the appended claims.

## Claims

1. An apparatus for analyzing components of urine, comprising:
a toilet bowl (710) which has a recessed or flat-shaped urine collecting part (730);
a piping part (720) for guiding the urine collected from the urine collecting part (730); and
an analyzing part (740) for analyzing the components of the urine introduced through the piping part,
wherein the urine collecting part (730) is formed at an inner front side of the toilet bowl (710), the analyzing part comprises ATR (Attenuated Total Reflectance) (744), and the analyzing part is directly attached to the toilet bowl.

2. The apparatus for analyzing components of urine as set forth in claim 1, wherein the piping part comprises:
an input pipe (721) for guiding the urine and a reference material measuring a reference value to the ATR (Attenuated Total Reflectance) (744); and
an output pipe (722) for discharging the reference material and the urine from the ATR.

3. The apparatus for analyzing components of urine as set forth in claim 2, wherein the input pipe comprises:
a solenoid valve (723) for controlling the reference material and the urine guided to the ATR;
at least one cartridge (726-728) for supplying the reference material; and
a cleaning tank for supplying a cleaning solution to clean the input pipe and the ATR.

4. An apparatus for analyzing components of urine, comprising:
a toilet bowl (710) which has a recessed or flat-shaped urine collecting part (730); and
an analyzing part (740) for analyzing the components of the urine collected at the urine collecting part (730),
wherein the urine collecting part (730) is formed at an inner front side of the toilet bowl (710), the analyzing part comprises ATR, and the analyzing part (740) is directly attached to the toilet bowl (710).

5. The apparatus for analyzing components of urine as set forth in claim 4, wherein the urine collecting part is horizontally or inclinedly formed at an inner side of the toilet bowl.

6. The apparatus for analyzing components of urine as set forth in claim 4 or 5, wherein the urine collecting part is formed into a hemispherical surface, and a hole is formed at a center portion of the hemispherical surface.

7. The apparatus for analyzing components of urine as set forth in any of claims 4 to 6, wherein the analyzing part comprises a prism (733), and an upper surface of the prism is formed into a curved shape and coupled to the hole, or formed into a fat shape and coupled to the hole, or the upper surface of the prism is attached under the hole.

8. The apparatus for analyzing components of urine as set forth in any of claims 4 to 7, wherein the urine analyzing apparatus further comprises a cleaning solution supplying part (712) which is disposed at a higher position than the urine collecting part so as to clean the urine collecting part.

9. The apparatus for analyzing components of urine as set forth in any of claims 4 to 8, wherein the urine analyzing apparatus further comprises an air injecting part (711) which is disposed at a higher position than the urine collecting part (730) so as to dry the urine collecting part (730).

10. The apparatus for analyzing components of urine as set forth in claim 1 or 4, wherein the analyzing part comprises:
a light source (741) for generating light;
a monochromator (742) for generating monochromatic light using the light;
a reflecting mirror (743) for guiding the monochromatic light to the ATR;
a detector (746) for detecting the monochromatic light passed through the ATR;
a controller (747) for measuring a property of the monochromatic light detected by the detector (746) and also controlling the light source (741), the monochromator (742), the reflecting mirror (743) and the detector (746).

11. The apparatus for analyzing components of urine as set forth in claim 10, wherein the monochromatic light has a wavelength of 1000 ∼ 15000nm.

12. The apparatus for analyzing components of urine as set forth in claim 10, wherein the analyzing part is made smaller by using MEMS (Micro Electro Mechanical Systems) technology.

13. A method of analyzing components of urine, comprising the steps of:
measuring a spectrum of a reference material introduced through a urine collecting part (730);
measuring an absorption spectrum of the urine introduced through the urine collecting part (730);
obtaining a calibration curve which indicates correlation between the absorption spectrum and a standard value which is generated by previously measuring each component of the urine;
estimating an amount of each component contained in the urine by using the calibration curve,
wherein the urine collecting part (730) is formed at an inner front side of a toilet bowl (710), the spectrum of the reference material and the spectrum of the urine are measured by using ATR, and the ATR is directly attached to the toilet bowl.

14. The method of analyzing components of urine as set forth in claim 13, wherein the spectrum of the reference material and the spectrum of the urine are measured by using monochromatic light introduced to the ATR.

15. The method of analyzing components of urine as set forth in claim 14, wherein monochromatic light has a wavelength of 1000 ∼ 15000nm.

16. The method of analyzing components of urine as set forth in any of claims 13 to 15, further comprising the step of cleaning the urine collecting part using a cleaning solution after measuring the absorption spectrum of the urine.

17. The method of analyzing components of urine as set forth in claim 16, wherein the cleaning solution and the reference material are the same material.

18. The method of analyzing components of urine as set forth in claim 16 or 17, further comprising the step of drying the urine collecting part (730) using an air injecting part which is disposed at a higher position than the urine collecting part.

19. A health examination system which comprises a bidet device disposed at a rear side of a toilet bowl and a body fat measuring apparatus (600) coupled with the toilet bowl,
wherein the body fat measuring apparatus comprises:
a handle disposed at left and right sides of the toilet bowl;
eight electrodes which are respectively provided at four contact points in pairs, and
wherein the two out of the four contact points are disposed at an upper surface of a seat portion of the toilet bowl, which is contacted with user's hips or femoral region, and the other two contact points are disposed at the handles.

20. The health examination system as set forth in claim 19, wherein each of the contact points comprises a voltage electrode and a current electrode, and the handle is disposed to be inserted into a side portion of the toilet bowl, and also a cover is further provided thereon so as to prevent water from being contacted.

21. The health examination system as set forth in claim 19, further comprising a urine analyzing apparatus for measuring components contained in urine, wherein ATR is directly attached to the toilet bowl.

22. A health examination system, comprising:
a bidet device which is disposed at a rear side of a toilet bowl;
a weight measuring apparatus for measuring user's weight using a plurality of load cells disposed under a seat portion of the toilet bowl; and
a urine analyzing apparatus for measuring components contained in urine using ATR,
wherein the ATR is directly attached to the toilet bowl.

23. The health examination system as set forth in claim 21 or claim 22, further comprising a blood pressure measuring apparatus for measuring user's blood pressure; and a fingerprint recognition apparatus for identifying a user of the urine analyzing apparatus by recognizing user's fingerprint,
wherein the blood pressure measuring apparatus and the fingerprint recognition apparatus are disposed at an arm rest on which user's arm is rested, and the user can simultaneously perform the fingerprint recognizing and the blood pressure measuring, while being seated on the toilet bowl.

24. The health examination system as set forth in claim 23, further comprising a monitor for displaying at least one of urine analyzing information measured by the urine analyzing apparatus, weight information measured by the weight measuring apparatus, fingerprint information recognized by the fingerprint recognizing apparatus, blood pressure information measured by the blood pressure measuring apparatus and body fat information measured by the body fat measuring apparatus,
wherein the monitor is disposed at the arm rest.

25. The health examination system as set forth in claim 24, further comprising an agent injecting apparatus for supplying an agent used in the health examination system,
wherein the agent injecting apparatus is inclinedly disposed at a rear side of the toilet bowl, and also the agent injecting apparatus is connected with the bidet device.

26. The health examination system as set forth in claim 24 or 25, wherein at least one of the urine analyzing information, the weight information, the fingerprint information, the blood pressure information and the body fat information can be transferred through Internet or Ethernet, and also analyzed results using the transferred information can be received.

27. A health examination system which comprises a bidet device disposed at a rear side of a toilet bowl and an electrocardiogram measuring apparatus coupled with the toilet bowl,
wherein the electrocardiogram measuring apparatus comprises:
two contact points disposed at left and right handles of the toilet bowl; and
two contact points disposed at an upper surface of the toilet bowl, which is contacted with user's hips or femoral region,
wherein each contact point has two electrodes, and the electrocardiogram measuring apparatus measures an electrocardiogram by transmitting an induced current through an user of the health examination system using the eight electrodes disposed at the four contact points and then measuring potential differences among the electrodes.

28. The health examination system as set forth in claim 27, wherein the electrocardiogram measuring apparatus obtains a potential difference among left and right hands and left femoral region by measuring a potential difference between left and right hands using a first induced current, a potential difference between left femoral region and right hand using a second induced current and a potential difference between left femoral region and left hand using a third induced current, and also the electrocardiogram measuring apparatus obtains a potential difference among left and right hands and right femoral region by measuring a potential difference between left and right hands using a fourth induced current, a potential difference between right femoral region and right hand using a fifth induced current and a potential difference between right femoral region and left hand using a sixth induced current, and then finally obtains the electrocardiogram using an average of the measured results.

29. The health examination system as set forth in claim 27 or 28, further comprising a urine analyzing apparatus for measuring components contained in the urine using ATR,
wherein the ATR is directly attached to the toilet bowl.
